(19) Europäisches Patentamt
European Patent Office
Office européen des brevets

(11) Numéro de publication : **0 228 387**
**B1**

(12) # FASCICULE DE BREVET EUROPÉEN

(45) Date de publication du fascicule du brevet : **05.04.89**

(51) Int. Cl.⁴ : **A 61 L 33/00**

(21) Numéro de dépôt : **86903200.3**

(22) Date de dépôt : **03.07.86**

(86) Numéro de dépôt international : **PCT/CH 86/00093**

(87) Numéro de publication internationale : **WO/8700060 (15.01.87 Gazette 87/01)**

(54) **SUBSTRAT DONT LA SURFACE PRESENTE UNE ACTIVITE ANTITHROMBOGENIQUE.**

(30) Priorité : **08.07.85 CH 2954/85**

(43) Date de publication de la demande : **15.07.87 Bulletin 87/29**

(45) Mention de la délivrance du brevet : **05.04.89 Bulletin 89/14**

(84) Etats contractants désignés : **BE CH FR GB IT LI NL SE**

(56) Documents cités :
**EP-A- 0 086 186**
**FR-A- 2 187 849**
**US-A- 4 521 564**
**US-A- 4 526 714**

(73) Titulaire : **BATTELLE MEMORIAL INSTITUTE**
**7 route de Drize**
**CH-1227 Carouge/Genève (CH)**

(72) Inventeur : **BICHON, Daniel**
**16, rue de Vallard**
**F-74240 Gaillard (FR)**
Inventeur : **SCHNEIDER, Michel**
**Domaine du Moulin**
**34, route d Annecy CH-1256 Troinex (CH)**
Inventeur : **GUILLOT, Christian**
**Les Eplanes Le Chable Beaumont**
**F-74160 Saint Julien en Genevois (FR)**

(74) Mandataire : **Dousse, Blasco et al**
**7, route de Drize**
**CH-1227 Carouge/Genève (CH)**

**EP 0 228 387 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

**Description**

La présente invention concerne un substrat dont la surface est antithrombogénique en raison de la présence, lié à celle-ci, d'un film adhérent de copolymère polyoléfinique comportant, fixés de manière covalente et distribués suivant un ordre aléatoire sur la chaîne principale, des groupes latéraux carboxyliques et des groupes de formule

$$—CONH—(CH_2)_n—NH—CH_2R$$

où R est l'héparine ou un fragment antithrombogéniquement actif dérivé de l'héparine par dépolymérisation de celle-ci, et n est un nombre entier compris entre 2 et 12.

Le présent copolymère peut également contenir, comme groupes latéraux, des groupes $—CONH—(CH_2)—_n—COOH$ où n vaut de 1 à 12.

Le film copolymère de l'invention présente donc des propriétés anti-coagulantes vis-à-vis du sang et convient particulièrement pour revêtir des substrats de natures diverses, notamment les plastiques utilisés dans les dispositifs de transfusion sanguines.

Les groupes —COOH libres du présent copolymère sont en quantité suffisante stœchiométriquement, et même en excès, pour neutraliser, si besoin est, la basicité résultant de la présence dans le présent copolymère de fonctions amines et ainsi améliorer l'hémocompatibilité de la surface revêtue du film antithrombogénique (voir, par exemple J. S. BYCK et al., Soc. Plast. Eng. Techn. Pap. 21, 563-566 (1975). Par ailleurs, de par le procédé de fabrication décrit ci-après, le présent film est pratiquement dépourvu de groupes amino primaires libres, ce qui lui confère un avantage supplémentaire sur les polymères anticoagulants de l'état de la technique. En effet, le procédé de base utilisé pour fabriquer ceux-ci consiste à fixer, sur un polymère comprenant des groupes amino libres, un fragment de dépolymérisation de l'héparine comportant une fonction réactive vis-à-vis de ces groupes amino libres, notamment des groupes aldéhydiques, par une réaction de SCHIFF suivie d'une réduction de la liaison —N = C ainsi obtenue. Or, comme la formation des bases de SCHIFF n'est pas quantitative, le polymère à propriétés antithrombogénique résultant du procédé ci-dessus contient des fonctions $—NH_2$ libres qui exercent une activité perturbatrice sur l'activité antithrombine de l'héparine liée.

On trouvera, dans les références suivantes, des détails sur les films dont la surface présente des propriétés anti-coagulantes connus de par l'état de la technique : EP-A-86 187 EP-A-86 186 M. FUNAHASHI et al, Analytical Biochemistry 126, 414-421 (1982) ; P. OLSSON et al, Annals of the New-York Academy of Sciences 1984, pp. 525-535 , EP-A-98 814 , EP-A-92 928. On notera tout particulièrement les documents suivants issus de la recherche effectuée par l'Office des Brevets de Vienne.

Le document US-A-3 673 612 (MIT) divulgue des compositions de polymères contenant de l'héparine. Dans ces compositions, l'héparine est liée au squelette du polymère par les liaisons acétal ou hémiacétal.

Le document US-A-4 239 664 (RESEARCH CORP) divulgue un polymère de polyvinyl-pyrrolidone et d'héparine dans lequel la liaison entre la liaison entre les chaînons du polymère et l'héparine est un groupe imino. Ce polymère présente des propriétés antithrombogéniques et est utilisable pour la fabrication de prothèses et dispositifs médicaux en contact avec le sang de patients.

Le document EP-A-46 828 (TEIJIN) divulgue l'esthérification de l'héparine par des dérivés acryliques ou méthacryliques et le greffage de l'héparine ainsi estérifiée sur des surfaces polymériques, ces surfaces étant celles d'appareils biomédicaux, tels que cathéters, conduits de transfusion, membranes de dialyse, composants de pompes, etc. Le greffage est effectué sous irradiation par ultra-violet, des faisceaux électroniques ou des rayons $\gamma$. Ce document divulgue encore l'héparinisation de matières plastiques dont la charpente comporte des groupes halogénures d'acide ou anhydride d'acide, la réaction d'héparinisation comprenant également une estérification.

Le document JP-82 39 851 (TERUMO) divulgue la quaternisation du polyméthacrylate de N,N-diméthylaminoéthyle et la fixation, sur ce polymère, d'héparine.

Le document JP-82-18 705 (GIJUTSUIN) divulgue la préparation d'un matériau anti-coagulant par réaction de l'acrylonitrile avec le méthacrylate de glycidyle puis fixation, sur le polymère ainsi obtenu, d'héparine. Un tel polymère, quoique se rapprochant en principe de celui de l'invention, comporte cependant une ossature bien différente et ne présente pas de groupe carboxylique libre.

De plus, dans le cas où le présent copolymère contient, en addition, des groupes $—CONH(CH_2)_n—COOH$, ces derniers contribuent, par leur adsorption de l'albumine du plasma, à réduire l'adsorption du fibrinogène, ce qui diminue encore la tendance que le sang pourrait avoir de coaguler au contact d'une paroi revêtue d'un tel copolymère. Le film anti-coagulant recouvrant le substrat de l'invention présente donc des avantages significatifs sur les produits connus correspondants. Parmi les divers articles auxquels conviennent le substrat de l'invention, on cite les cathéters, les tuyaux souples de nylon, de polyéthylène, de polyesters ou de polyuréthanne utilisés en chirurgie, les récipients pour conserver le sang, les pompes et organes de pompes et tous autres éléments pouvant être introduits dans le système circulatoire d'un patient et mis en contact avec le sang à l'extérieur ou à l'intérieur du corps de celui-ci.

Parmi les diverses compositions possibles pour le copolymère constituant le film adhésif utilisé dans

l'invention, on peut citer les photocopolymères à base d'acide acrylique, de méthacrylate de diméthylaminoéthyle (DMAEMA), de diméthylacrylamide et d'un co-monomère susceptible de fixer un fragment d'héparine, notamment l'acrylate de N-hydroxysuccinimide (ANHS). De tels copolymères ont l'avantage d'adhérer fortement à la plupart des surfaces plastiques habituelles et d'être obtenues très facilement et rapidement par irradiation, en présence d'un photoinitiateur, d'un film de composition approprié contenant les monomères correspondants. En plus des monomères précités, la présente composition peut également contenir d'autres monomères acryliques, notamment les acrylates d'alcoyles tels qu'isopropyle, butyle, amyle et autres, l'acrylamide, et des prépolymères acryliques tels que ceux cités dans le document US-A-4 451 568, de tels produits, de poids moléculaire relativement élevé, ayant une influence sur la viscosité du mélange de monomères. La quantité d'acide acrylique de la composition ci-dessus est stœchiométriquement supérieure à celle du DMAEMA afin que les groupes —COOH soient toujours en excédent par rapport aux groupes diméthylamino. Ainsi, pour une mole de DMAEMA, on utilise de 1,01 à 2 moles d'acide acrylique.

Le radical R dénommé fragment d'héparine est dérivé des produits de dépolymérisation de l'héparine, par exemple par scission désaminative de l'héparine, tels que décrits par OLSSON (voir la référence susmentionnée).

Pour préparer le substrat suivant l'invention, on revêt par les moyens habituels (pinceau, trempé, pulvérisation) un article sélectionné d'une couche mince d'un mélange de monomères photopolymérisable tels qu'évoqués plus haut et contenant un photoinitiateur, on procède à sa photopolymérisation par irradiation suivant les moyens habituels ; puis, lorsque le film a acquis le degré de durcissement voulu, on le lie avec le dérivé d'héparine par l'intermédiaire d'une diamine de pontage de formule $H_2N(CH_2)_n—NH_2—$ de manière à réaliser un chaînon de pontage (désigné dans ce qui précède par la formule $—NH(CH_2)_n—NH—$), ce chaînon permettant d'assurer la liaison entre le copolymère et le fragment d'héparine.

De préférence, on réalise ce chaînon par réaction de cette diamine aliphatique avec le groupement —CHO lié au fragment d'héparine tel qu'il résulte de la fragmentation décrite par OLSSON (voir la référence précitée). En effet, suivant cet auteur, la réaction de l'héparine avec l'acide nitreux convertit le résidu 2-amino-2-desoxy-D-glucopyrannosyle en un résidu aldéhydique de 2,5-anhydro-D-mannose possédant, tout comme l'héparine de départ, des propriétés anti-coagulantes. La réaction de couplage ci-dessus fournit donc une base de SCHIFF qu'on réduit ensuite (par exemple par le cyanoborohydrure de sodium), de manière à obtenir un substituant $R—CH_2—NH(CH_2)_n—NH_2$. On fait alors réagir ce dernier avec 'e film de polymère sec contenant, comme on l'a vu plus haut, copolymérisé avec les autres mono.nères, un groupement susceptible de se lier à la fonction amine libre du bras de pontage ménagé sur le fragment d'héparine. Ce groupement, notamment le reste N-hydroxysuccinimide (NHS), très labile, réagit comme suit :

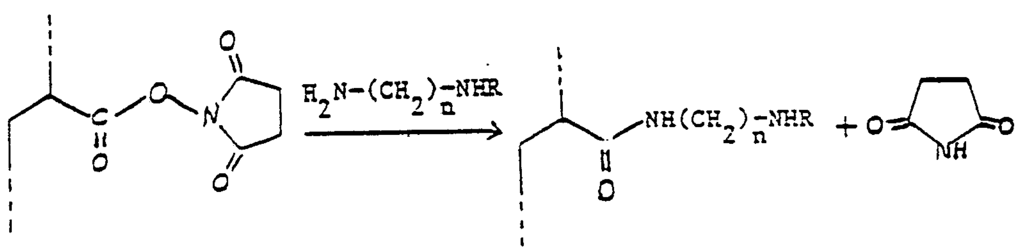


Cette opération a donc pour effet de fixer le fragment d'héparine R sur le film de copolymère par l'intermédiaire du chaînon de pontage précité. Pour celui-ci, on utilise volontiers comme diamine l'hexaméthylène diamine, mais, bien entendu, toute autre diamine comprise entre les limites indiquées peut convenir, notamment l'éthylène diamine, la propylène diamine, la butylène diamine, etc...

Lorsque la fixation du groupe hépariné est achevée, on neutralise les fonctions NHS restantes avec un réactif approprié, par exemple de l'éthanolamine, de la glycine ou d'autres acides aminoalcoyl carboxyliques, ce qui présente l'avantage, dans ce cas, de fournir des chaînes latérales pourvues de groupes carboxyliques. On peut également, pour réaliser le présent film antithrombogénique, procéder en sens inverse, c'est-à-dire commencer par fixer la diamine sur le copolymère et, ensuite, faire réagir sur le copolymère aminé le fragment d'héparine par son groupe aldéhydique, réaction qu'on fait suivre d'une réduction par le cyanoborohydrure de sodium. Dans un tel cas, la présence éventuelle de groupements $—NH_2$ libres (n'ayant pas réagi avec l'aldéhyde) n'exerce qu'un effet négatif peu prononcé en raison de la présence des groupes —COOH en excédent.

Comme photoinitiateurs de polymérisation, on peut utiliser la benzophénone, ses dérivés et autres photoinitiateurs usuels, tels que ceux décrits dans le document EP-A-69133.

Les exemples qui suivent illustrent l'invention.

Exemple 1

a) Préparation d'un substrat revêtu d'un film aux propriétés antithrombogéniques.

On a sélectionné des plaquettes de PVC munies d'une alvéole ou puits de titration (Microtiter plate FALCON) et on les a revêtues d'un film de mélange de monomères contenant les ingrédients suivants :

| | |
|---|---|
| Diméthylacrylamide | 45 g |
| Acide acrylique | 15 g |
| DMAEMA | 25 g |
| ANHS | 10 g |
| p-36 | 5 g |
| Total | 100 g |

Le produit dénommé « p-36 » est un photoinitiateur de structure benzophénonique fourni par la Société U.C.B. (Belgique).

On a irradié ces plaquettes 5 min sous azote au moyen d'une lampe UV produisant un flux de 30 W/cm à 30 cm de distance ce qui a fourni, sur les parois des puits, un film réactif d'environ 10-50 um d'épaisseur.

Par ailleurs, on a procédé à la désamination d'un échantillon d'héparine suivant J.E. SHIVELY et al., Biochemistry 15 (18), 3392 (1976) : on a ajouté à une solution de 0,1 g d'héparine dans 4,5 ml de solution aqueuse d'acide citrique (0,2 M, pH 4) 0,5 ml d'une solution aqueuse 1 M de nitrite de sodium. Après 30 min à température ambiante, on a ajusté le pH à 7 (solution NaCH 0,1 N) et on a procédé à une dialyse dans de l'eau distillée (membrane SPECTRAPOR, porosité : jusqu'à poids moléculaire 1000).

On a ajouté à la solution dialysée de fragments d'héparine aldéhydiques (Hep-CHO) une quantité suffisante d'hexaméthylène diamine (HMD) (solution aqueuse 3 M) pour réaliser une solution finale 0,8 M. Après 3 h d'incubation à température ambiante, on a procédé à la réduction de l'imine par adjonction de 1 ml de solution aqueuse 1 M de $NaCNBH_3$ et après 2 h, on a procédé à une nouvelle dialyse dans l'eau pure, à 4 °C avec une membrane identique à celle mentionnée ci-dessus. Finalement, on a porté le volume du produit dialysé (HMD-Hep) à 3,5 ml par mise en contact avec de l'Aquacide (carboxyméthylcellulose sodique). On a ensuite dilué cette solution à 28 mg/ml au moyen d'un tampon phosphate 0,04 M, pH8.

On a introduit dans les puits recouverts d'un revêtement réactif comme décrit plus haut, 200 ul de la solution HMD-Hep et on a laissé 2 jours en incubation. On a rincé par de l'eau distillée, après quoi on a introduit 200 ul d'une solution aqueuse 1 M de glycine afin de neutraliser l'excédent non transformé des groupes hydroxysuccinimide en groupes —COOH. On a ensuite procédé à un rinçage sous agitation avec de l'eau d'abord, puis du PBS (Phosphate Buffered Saline : 0,15 M NaCl, 10 mM $Na_2HPO_4$, pH 7,4). Les cuvettes ainsi préparées ont été étiquetées « Test 1 ».

b) Mesure de la capacité de blocage de la thrombine.

La mesure de l'activité antithrombine de l'héparine est basée sur le fait que, en combinaison avec l'antithrombine III, elle bloque l'activité de la thrombine. La mesure est donc basée sur l'addition d'un excès connu de thrombine à la solution à tester et la détermination de la quantité résiduelle de celle-ci enzymatiquement active.

Les réactifs utilisés sont tirés du « kit » BOEHRINGER (Mannheim) pour mesurer l'héparine en faibles doses. L'excès de thrombine est mesuré par son effet de catalyse sur l'hydrolyse d'un polypeptide p-nitroanilidé (CHROMOZYM TH), laquelle libère la p-nitroaniline qui est titrée colorimétriquement.

Le réactif « thrombine » est préparé à partir des ingrédients suivants : 10 ml de tampon Tris HCl 200 mM (pH 8,1), 25 mM NaCl, 6,5 IU/ml d'aprotinine, 100 ul de solution de thrombine (2,2 U/ml), 300 ul de solution d'antithrombine III SIGMA (10 U/ml).

Pour la mesure proprement dite, on ajoute 100 ul du réactif ci-dessus au puits à tester et après un temps donné d'incubation (par exemple 3 minutes) on ajoute une quantité constante du réactif CHROMOZYM-TH et, après dilution à un volume donné, on mesure l'intensité de la couleur à 405 nm, celle-ci étant proportionnelle à la quantité de thrombine non inhibée. Les résultats obtenus (test 1) figurent plus loin.

c) Mesure du « temps de thrombine » (Thrombin time).

Pour cette mesure, on a utilisé les réactifs du « kit » de Boehringer : « Thrombin reagents ».

Dans un des puits « héparinisé » suivant le procédé décrit plus haut (a), on a ajouté 100 ul de plasma « pauvre en plaquettes » (Platelet Poor Plasma, PPP) et on a agité doucement 2 min à 37 °C au moyen d'un barreau magnétique. On a ajouté ensuite 100 ul du réactif « Thrombin reagent » et on a mesuré le temps au bout duquel apparaît un coagulum blanc (de fibrine). Les résultats figurent au tableau ci-après à l'Exemple 2.

Exemple 2

On a sélectionné des plaquettes munies de cuvettes de titration (puits) revêtues de photopolymère irradié telles que décrites au début du paragraphe (a) de l'Exemple 1. On a traité ces cuvettes 1 nuit, avec une solution 1 M de HMD (Test 2). Par ailleurs, afin d'obtenir un témoin préparé suivant la technique antérieure, on a traité une cuvette avec une solution de 10 % en poids de polyéthylène imine (échantillon ii). On a également préparé un témoin non activé (iii) en neutralisant simplement les groupes réactifs NHS du film avec de l'éthanolamine.

On a ensuite utilisé, pour les essais (cuvettes Test 2, ii et iii), la solution résultant de la désamination de l'héparine décrite plus haut, après dialyse mais avant addition de HMD, qu'on a préalablement diluée à environ 5 mg/l dans le tampon phosphate 0,1 M, pH 8. On a introduit 200 ul de cette solution dans chacun des échantillons et, après 1 nuit d'incubation à température ambiante, on a ajouté un excès de solution 1 M de cyanoborohydrure de sodium pour réduire la liaison —N=C, et on a rincé à l'eau, au PBS, puis au sérum. En ce qui concerne le témoin (iii), le film ne contenant plus de sites de fixation, on suppose que les fragments d'héparine n'ayant pu se fixer ont été éliminés au rinçage.

Les échantillons Test 2, ii et iii ont été testés, tout comme l'échantillon de l'Exemple 1 (Test 1), les résultats figurant au Tableau ci-dessous.

| Echantillon | Activité antithrombine (U) | Temps de thrombine (sec) |
|---|---|---|
| Test 1 | 0,12 | > 300 |
| Test 2 | 0,10 | > 300 |
| ii | 0,01 | 22 |
| iii | 0,03 | 28 |

On voit, de par ces résultats, que le substrat de l'invention (Test 1 et 2) présente une activité antithrombine bien supérieure à celle d'un échantillon (ii) préparé suivant l'art antérieur. Par ailleurs, on constate que les groupes amino résiduels de l'échantillon (ii) ont une action inhibitrice (c.f. avec le témoin inactif iii) sur l'activité antithrombine de l'héparine fixée.

**Revendications**

1. Substrat comportant, sur au moins une partie de sa surface, un film adhérent de propriétés antithrombogéniques, caractérisé par le fait que ce film est constitué d'un copolymère oléfinique comportant, fixés de manière covalente sur la chaîne principale et distribués dans un ordre aléatoire, des groupes latéraux carboxyliques et des groupes de formule $—CONH—(CH_2)_n—NH—CH_2R$ (I) où R est une molécule d'héparine ou un fragment de dépolymérisation de celle-ci et n est un entier compris entre 2 et 12.

2. Substrat suivant la revendication 1, caractérisé par le fait que le copolymère comporte des segments dérivés des monomères suivants : acide acrylique, méthacrylate de N-diméthylaminoéthyle, diméthylacrylamide et dérivés amido-acrylique ou le groupe amido est celui représenté par la formule 1.

3. Substrats suivant la revendicat:on 1, caractérisé par le fait en addition, des groupes latéraux de formule $—CONH(CH_2)_n—COOH$ ou n est compris entre 1 et 12.

4. Substrat suivant la revendication 1, caractérisé par le fait que R est un fragment de désamination par $HNO_2$ de l'héparine.

5. Procédé de préparation de substrat suivant la revendication 1, caractérisé par les étapes suivantes :

a) on prépare un mélange photopolymérisable comprenant de l'acide acrylique, du méthacrylate de N-diméthylaminoéthyle, du diméthylacrylamide, un acrylate d'un composé hydroxylabile, tel que la N-hydroxy succinimide ou un autre monomère susceptible de fixer un dérivé héparinique, par exemple un acrylate de 2,3-époxypropyle et un photoinitiateur ;

b) on dépose une couche de ce mélange sur la surface d'un substrat et on procède à son durcissement par irradiation ;

c) on soumet de l'héparine par traitement au $HNO_2$, à une dépolymérisation et désamination en fragments hépariniques actifs porteurs d'une fonction aldéhydique (R—CHO) et

d) on couple ces fragments au film obtenu selon (b) au moyen d'une diamine de pontage de formule $H_2N—(CH_2)_n—NH_2$, l'une des extrémités de celle-ci formant une liaison amide par réaction avec ledit composé hydroxylabile et l'autre extrémité formant une liaison —NH—CH R par réaction avec le

groupe aldéhyde, suivie d'une réduction de la base SCHIFF intermédiaire obtenue.

6. Procédé suivant la revendication 5, caractérisé par le fait qu'on utilise, dans le mélange de l'étape (a), au moins 1,01 équivalent molaire d'acide acrylique par équivalent de méthacrylate de diméthylaminoéthyle.

7. Utilisation du substrat suivant la revendication 1, à la fabrication d'éléments d'appareils chirurgicaux entrant en contact avec le sang, tels que cathéters, tuyaux souples pour la transfusion, récipients, pompes.

## Claims

1. Substrate having on at least a portion of its surface an adhesive film with antithrombogenic properties, characterized in that this film is constituted of an olefinic copolymer comprising, covalently bound to the main chain and distributed randomly thereon, carboxylic side groups and groups of formula $—CONH—(CH_2)_n—NH—CH_2R$ (I) wherein R is a heparin molecule or a depolymerization fragment thereof and n is a integer from 2 to 12.

2. Substrate according to claim 1, characterized in that the copolymer comprises segments derived from the following monomers : acrylic acid, N,N-dimethylaminoethyl methacrylate, dimethylacrylamide, and amido-acrylic derivatives in which the amide group is that represented by formula I.

3. Substrate according to claim 1, further characterized by side groups of formula $—CONH—(CH_2)_n—COOH$ wherein n is from 1 to 12.

4. Substrate according to claim 1, characterized in that R is a fragment issued from the $HNO_2$ deamination of heparin.

5. Method of preparation of the substrate according to claim 1, characterized by the following steps :

a) a photopolymerizable mixture is prepared comprising acrylic acid, N,N-dimethylaminoethyl methacrylate, dimethylacrylamide, an acrylate of a compound with a labile hydroxy such as N-hydroxysuccinimide or another monomer capable of binding a heparin derivative, for instance 2,3-epoxypropyl acrylate, and a photoinitiator ;

b) a layer of this mixture is deposited on the surface of a substrate and its curing by irradiation is carried out ;

c) heparin is subjected, under treatment by $HNO_2$, to a depolymerization and deamination into active heparin fragments carrying an aldehyde function (R—CHO) ; and

d) these fragments are coupled to the film obtained according to (b) by means of a bridging diamine of formula $H_2N—(CH_2)_n—NH_2$, one end thereof forming an amide bond by the reaction with said compound having a leaving hydroxy, and the other end forming a $—NH—CH_2—R$ by the reaction with the aldehyde group and subsequent reduction of the intermediate SCHIFF base obtained.

6. Method according to claim 5, characterized in that there is used, in the mixture of step (a), at least 1.01 mole equivalent of acrylic acid per equivalent of dimethylaminoethyl methacrylate.

7. The use of the substrate according to claim 1, for the manufacture of components of surgical apparatuses to be put into contact with blood, e. g. catheters, flexible transfusion tubing, containers, pumps, etc.

## Patentansprüche

1. Substrat, welches zumindest auf einem Teil seiner Oberfläche einen anhaftenden Film mit antithrombogenen Eigenschaften aufweist, dadurch gekennzeichnet, daß dieser Film aus einem Olefin-Copolymer besteht, welches an der Hauptkette, in einer zufälligen Reihenfolge verteilt und kovalent gebunden, seitliche Carboxylgruppen und Gruppen der Formel $—CONH—(CH_2)_n—NH—CH_2R$ (I) aufweist, wobei R ein Heparinmolekül oder ein Fragment seiner Depolimerisation ist, und wobei n eine ganze Zahl zwischen 2 und 12 ist.

2. Substrat nach Anspruch 1, dadurch gekennzeichnet, daß das Copolymer Abschnitte aufweist, die von folgenden Monomeren abgeleitet sind : Acrylsäure, N-Dimethylaminoäthylmethacrylat, Dimethylacrylamide und Amido-Acrylderivate, wobei die Amidogruppe jene der in der Formel 1 dargestellten ist.

3. Substrate gemäß Anspruch 1, dadurch gekennzeichnet, daß zusätzlich seitliche Gruppen der Formel $—CONH(CH_2)_n—COOH$ vorgesehen sind, wobei n zwischen 1 und 12 liegt.

4. Substrat nach Anspruch 1, dadurch gekennzeichnet, daß R ein von Heparin durch Desaminierung mittels $HNO_2$ erhaltenes Fragment ist.

5. Verfahren zur Herstellung des Substrates gemäß Anspruch 1, gekennzeichnet durch die folgenden Stufen :

a) man stellt eine photopolymerisierbare Mischung her, die Acrylsäure, N-Dimethylaminoäthylmethacrylat, Dimethylacrylamid, ein Acrylat einer hydroxyabgebenden Verbindung, wie N-Hydroxysuccinimid oder ein anderes Monomer, das zur Bindung eines Heparinderivates fähig ist, beispielsweise ein 2,3-Epoxypropylacrylat, und einen Photoinitiator enthält ;

b) man gibt eine Schicht dieser Mischung auf die Oberfläche eines Substrats und härtet sie durch

Bestrahlung ;

c) man unterwirft das Heparin mittels Behandlung mit $HNO_2$ einer Depolymerisation und Desamination in aktive Heparinfragmente, die eine Aldehyd-Funktionsgruppe (R—CHO) tragen und

d) man kuppelt diese Fragmente mit dem gemäß (b) erhaltenen Film mittels eines Überbrückungsdiamins der Formel $H_2N—(CH_2)_n—NH_2$, wobei dessen einer Arm eine Amidbindung durch Reaktion mit der genannten hydroxyabspaltenden Verbindung und der andere Arm eine —NH—CH R-Bindung durch Reaktion mit der Aldehydgruppe bildet, gefolgt von einer Reduktion der als Zwischenprodukt erhaltenen Schiff'schen Base.

6. Verfahren nach Anspruch 5, dadurch gekennzeichnet, daß man in der Mischung der Stufe (a) zumindest 1,01 Mol Äquivalent Acrylsäure pro Äquivalent Dimethylaminoäthylmethacrylat verwendet.

7. Verwendung des Substrates gemäß Anspruch 1 zur Herstellung von Elementen chirurgischer Apparate, die mit Blut in Kontakt treten, wie z. B. Katheter, flexible Transfusionsschläuche, Behälter und Pumpen.